Europäisches Patentamt

European Patent Office

Office européen des brevets

(19)

(11) **EP 0 531 735 B1**

(12) **EUROPÄISCHE PATENTSCHRIFT**

(45) Veröffentlichungstag und Bekanntmachung des Hinweises auf die Patenterteilung:
**20.10.1999 Patentblatt 1999/42**

(51) Int. Cl.$^6$: **A61K 35/78**

(21) Anmeldenummer: **92113792.3**

(22) Anmeldetag: **13.08.1992**

(54) **Verwendung von Avena sativa L. zur Herstellung entzündungshemmender Arzneimittel**

Use of Avena sativa L. for the preparation of anti-inflammatory medicaments

Utilisation d'Avena sativa L. pour la préparation de médicaments antiinflammatoires

(84) Benannte Vertragsstaaten:
**CH DE FR GB IT LI NL**

(30) Priorität: **11.09.1991 AT 180891**

(43) Veröffentlichungstag der Anmeldung:
**17.03.1993 Patentblatt 1993/11**

(73) Patentinhaber:
**Heller, Wolfgang, Mag.**
**A-6800 Feldkirch (AT)**

(72) Erfinder: **Heller, Wolfgang, Mag.**
**A-6800 Feldkirch (AT)**

(74) Vertreter:
**Hefel, Herbert, Dipl.-Ing.**
**Egelseestrasse 65a**
**Postfach 61**
**6800 Feldkirch (AT)**

(56) Entgegenhaltungen:
**US-A- 4 175 124**

• **CAB ABSTRACTS ,ZUSAMMENFASSUNG Nr 0Q035- 03899,SAEED, S.A. ET AL.: PROSTAGLANDIN BIOSYNTHESIS IN EXTRACTS OF (AVENA SATIVA) SEEDS. SOCIETY TRANSACTIONS 1981. 9(5):444.SIEHE ZUSAMMENFASSUNG**

**Beschreibung**

Die Erfindung betrifft die Verwendung von Fraktionen aus Extrakten der Gesamtpflanze Avena sativa L. ohne Samen mit einem Molekulargewicht der Inhaltsstoffe kleiner als 1000 zur Herstellung von Arzneimitteln zur Hemmung der Biosynthese von Prostaglandinen sowie zur Behandlung von Erkrankungen, die ganz oder teilweise mit der Prostaglandin-Biosynthese zusammenhängen sowie zur Hemmung entzündlicher Prozesse.

Bekannt ist es, Haferschleim bei Gastroenteritiden und Dyspepsie anzuwenden. Der Haferschleim entfaltet dabei eine mechanische Schutzfunktion durch Auskleiden der entzündeten Schleimhäute. Bei Haferschleim handelt es sich um eine Schleimdroge, die primär auf Polysacchariden basiert. Schleimdrogen, zu welchen ja Haferschleim gehört, entfalten nur eine örtliche Wirkung, nämlich: Sie wirken lokal reizmindernd, d.h. sie sind Antireizstoffe (keine Antiphlogistika im Sinne der modernen Pharmakologie); sie wirken absorbierend; sie wirken kühlend (Wasserdepot für die Haut). Bei Haferschleim (Schleimdrogen) handelt es sich nicht um Antiphlogistika im Sinne der Pharmakologie ("Lehrbuch der Pharmakognosie und Phytopharmazie" E. Steinegger- R. Hänsel, 4. Auflage, Seite 122/123). Antiphlogistika sind Stoffe, die entzündungshemmend wirken, wobei das Wort "Entzündung" hier im streng medizinischen Sinne verstanden werden soll und nicht in seinem sprachüblichen Gebrauch. Auf eben diesen Einsatz als Antiphlogistika zielt die Erfindung ab.

Im Gegensatz zu einer Reihe bekannter pflanzlicher, entzündungshemmender Mittel bzw. Antirheumatika besteht die vorliegende Erfindung ausschließlich aus dem Extrakt einer einzigen Pflanze. Dies entspricht der modernen Tendenz, übersichtlichere Zusammenstellungen mit geringer Anzahl an pflanzlichen Bestandteilen zu schaffen, die sich durch geringe unerwünschte Wirkungen auszeichnen und sich in der Hauptwirkung mit chemisch erzeugten, entzündungshemmenden Produkten vergleichen lassen. Die vorliegende Erfindung kommt dieser Forderung stark entgegen. Die entzündungshemmende Wirkung der erfindungsgemäßen Extrakte wird durch eine Reihe von Versuchsergebnissen dokumentiert (s.u.).

Aus Biochemical Society Transactions 9, 444 (1981) ist die Verwendung wässriger oder alkoholischer Extrakte von Samen der Pflanze Avena sativa zur Behandlung von Entzündungen bekannt. Es ist die Inhibierung der Prostaglandinsynthese durch wässrige oder alkoholische Extrakte dieser Samen beschrieben, wobei die durch Ultrafiltration gewonnene aktive Fraktion ein Molekulargewicht von 1.000 - 10.000 Dalton aufweist. Hier wird jedoch ausschließlich und allein auf den Samen von Avena sativa und auf das dadurch gewonnene Filtrat mit einem Molekulargewicht von größer als 1.000 verwiesen.

Da vor allem Prostaglandine (besonders Prostaglandin $E_2$ und $I_2$) am Entzündungsgeschehen im weitesten Sinn beteiligt sind, also Entzündungsmediatoren darstellen, wurde untersucht, ob Haferextrakte die Biosynthese von Prostaglandinen beeinflußt. Prostaglandine bzw. Eicosanoide (s.u.) entstehen aus meist Zellmembran-gebundenen Vorstufen, in erster Linie aus Arachidonsäure (Eicosatetraensäure: daher bezeichnet man die Gesamtheit der Metaboliten als "Eicosanoide"). Die Biosynthese von Prostaglandinen wird durch eine Reihe von körpereigenen Stoffen angeregt. Im vorliegenden Fall sind es vor allem Entzündungsmediatoren, wie z.B. das Polypeptid Bradykinin oder auch Histamin, Serotonin u.a.. Prinzipiell führt jede Störung der Zellmembran(z.B. auch durch UV-Strahlung, Verbrennung, Verletzung) zu einer massiven Steigerung der Bildung von Prostaglandinen. Die Biosynthese beginnt in den meisten Fällen durch die Aktivierung einer Phospholipase $A_2$, die als Schlüsselenzym bezeichnet werden kann. Dieses spaltet Arachidonsäure von Membran Phospholipiden ab. Die freie Arachidonsäure wird nun intrazellulär durch die Cyclooxygenase über labile Zwischenprodukte (sog. Endoperoxide $PGG_2$ und $PGH_2$) in Prostaglandine, Thromboxan $A_2$, Malondialdehyd HHT (eine Hydroxyfettsäure) und Sauerstoffradikale umgewandelt. Unter bestimmten Bedingungen, insbesondere bei allergischen Prozessen, entstehen aus Arachidonsäure z.B. in Leukozyten, Mastzellen der Lunge etc. durch die Lipoxygenase über labile Peroxyfettsäuren und Hydrooxyfettsäuren die sog. Leukotriene ($A_4$, $B_4$, $C_4$, $D_4$, $E_4$, $F_4$) und Lipoxine (A und B). Neben den erwähnten Prostaglandinen ist auch Leukotrien $B_4$ am Entzündungsgeschehen beteiligt. Die Leukotriene $C_4$, $D_4$ und auch $E_4$ spielen eher bei der Allergie (z.B. Asthma bronchiale) eine entscheidende Rolle.

Die Wirkung der Prostaglandine (bes. $E_2$) beim Entzündungsgeschehen ist vielfältig. Sie erweitern z.B. Blutgefäße, erhöhen die Kapillarpermeabilität und tragen initial zur Fieberentstehung bei. Ausgeprägter als der Eigeneffekt der Prostaglandine bei Entzündungen ist jedoch ihre Modulatorfunktion. Prostaglandin $E_2$ (und teilweise auch $PGI_2$) sensibilisiert "Schmerzrezeptoren" an sensorischen Nerven, d.h., es verstärkt die Schmerzwirkung körpereigener Stoffe, die Schmerzrezeptoren reizen und derart den sog. Entzündungsschmerz auslösen. Prostaglandin $E_2$ senkt also die Schmerzschwelle. Auch die typischen Entzündungszeichen (Erhöhung der Gefäßpermeabilität = Ödem, Erweiterung der Blutgefäße = Erythem und Wärmegefühl), die durch andere Entzündungsmediatoren verursacht werden (s.o.), unterliegen der verstärkenden Wirkung der Prostaglandine.

Abgesehen von mehreren physiologischen modul a torischen Funktionen der Prostaglandine (z.B. im Gefäßsystem oder bei der Geburt), gibt es eine Reihe weiterer pathophysiologischer Vorgänge, an denen diese Fettsäurederivate beteiligt sind. So fördert etwa eine erhöhte Prostaglandinbiosynthese im Darm die Wassersekretion und verursacht dadurch Diarrhoe - um nur ein Beispiel anzuführen.

Bis zum Beginn der 70er Jahre war unbekannt, auf welche Weise Acetylsalicylsäure ihre entzündungshemmende Wirkung ausübte. Dann stellte sich heraus, daß diese Substanz die Synthese der Prostaglandine hemmt , was sich letztlich als wichtigster Wirkungsmechanismus erwies. Diese Entdeckung führte letztlich zu allen oben erwähnten Erkenntnissen über die Bedeutung der Prostaglandine beim Entzündungsgeschehen. Alle heute bekannten sog. Nichtsteroidalen Antiphlogistika, die in der Rheumatherapie eingesetzt werden, wirken in erster Linie über Hemmung der Prostaglandin-Biosynthese oder genauer, über Hemmung der Cyclooxygenase.

Natürlich wird das überaus komplizierte System der Entzündungsvorgänge durch das Zusammenspiel mehrerer Entzündungsmediatoren bestimmt. Blockiert man jedoch die Synthese der Prostaglandine, kommt es zu einer Verringerung oder zu einem Verschwinden der Entzündungssymptome - je nach dem Ausmaß der Beteiligung der Prostaglandine. Aus diesem Grund kann die Prüfung einer Substanz auf entzündungshemmende Eigenschaften direkt durch deren Einfluß auf die Prostaglandin Biosynthese erfolgen.

Eine Methode, die lokale, entzündungshemmende Wirkung einer Substanz zu prüfen, stellt der Crotonöl-Test dar. Mit Aceton verdünntes Crotonöl führt auf der Haut zu einem Erythem. Dessen Entstehung wird durch lokal applizierte entzündungshemmende Stoffe gehemmt. Eine weitere, gut bekannte Methode zur Prüfung entzündungshemmender Stoffe ist das Carrageenan Pfotenödem der Ratte. Dieses stellt das experimentelle Modell einer akuten Entzündung dar. Das in kleinsten Mengen subplantar injizierte Carrageenan führt zu einer Schwellung der Pfote, also zu einem Ödem. Eine ganze Reihe bekannter entzündungshemmender Substanzen ist an diesem Modell wirksam. Das Modell erfaßt auch Stoffe, die nicht (nur) die Prostaglandin-Biosynthese hemmen. Carrageenan führt nämlich zur Freisetzung mehrerer Entzündungsmediatoren und nicht nur von Prostaglandinen. Ein Prinzip, welches die Volumszunahme des Carrageenan-induzierten Pfotenödems hemmt, kann dies folglich über Hemmung der Freisetzung und/oder Wirkung eines oder mehrerer bekannter oder unbekannter Entzündungsmediatoren verursachen.

Die vorliegende Erfindung erwies sich in mehreren Varianten (s.u.) als wirksam in allen drei oben beschriebenen Prüfungsmethoden. Ein Wirkstoff oder mehrere Wirkstoffe von Avena sativa L. hemmen die Prostaglandin-Biosynthese in vitro, verhindern bzw. verringern die Hautrötung bei lokaler Gabe im Crotonöl Test und hemmen die Entstehung des Pfotenödems nach oraler Gabe. Die Wirkstoffe sind vor allem in wäßrigen Auszügen von Avena sativa L. enthalten und hier wiederum, was die Hemmung der Prostaglandinbiosynthese betrifft, in der Gesamtpflanze ohne Samen. Die erfindungsgemäßen Auszüge eröffnen neue Einflußmöglichkeiten auf die Prostaglandin-Biosynthese und auf Entzündungen, die möglicherweise auch mit der Hemmung der Prostaglandin-Biosynthese in Zusammenhang stehen.

Die Verwendung der erfindungsgemäßen Produkte wird sich also in erster Linie auf die Blockade der Eicosanoid- bzw. Prostaglandin-Biosynthese sowie auf die Hemmung von Entzündungen richten. Die Auszüge können zur Herstellung von Arzneimitteln für die angeführten Anwendungsbereiche in Frage kommen, wobei lokale und orale Applikationen möglich sind.

Methoden

a) Herstellungsverfahren

Im folgenden Abschnitt werden verschiedene Herstellungsverfahren für wirksame Extrakte von Avena sativa L. beschrieben.

Beispiel 1 (für wässrige Extrakte)

Avena sativa L. 0,25 g, 0,8 g, 2 g, 5 g und 8 g werden mit 150 ml und 0,25 g bzw. 2,5 g mit 100 ml heißem Wasser übergossen und 10 Minuten unter mehrfachem Umrühren bedeckt stehen gelassen.

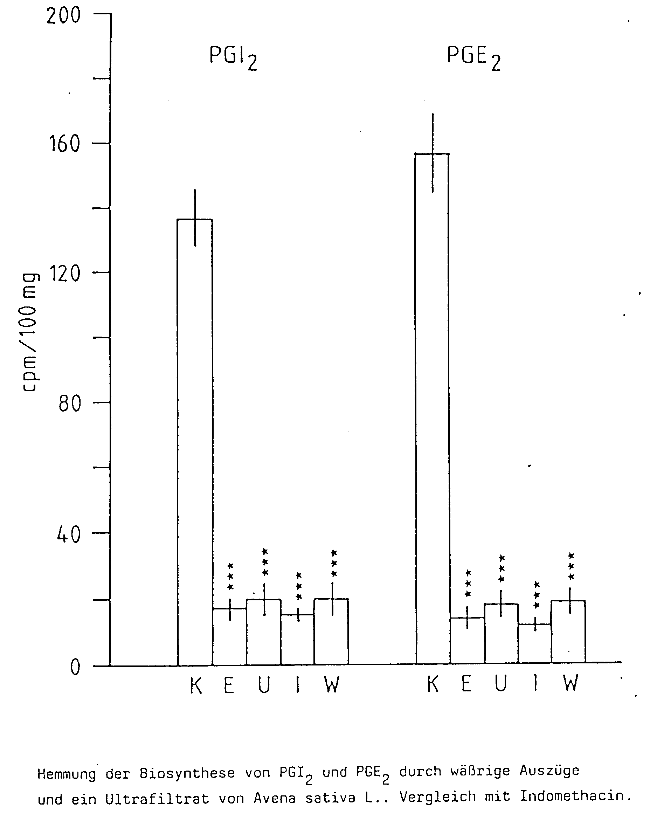

Nach der Filtration werden die für die Prüfung auf Hemmung der Prostaglandin-Biosynthese gedachten Extrakte mit Natriumchlorid auf Blutisotonie gebracht, wozu ein Osmometer verwendet wird. Die Extrakte für die lokale Anwendung zur einfacheren Auftragung mit Methylzellulose (Endkonzentration 1 %) versetzt. Die Extrakte für die orale Gabe zur Prüfung am Carrageenan Pfotenödem wurden im ursprünglichen Zustand belassen. Die Wirkungen dieser Extrakte sind in Abb. 1,2,4 dargestellt.

Für die Prüfung des Einflusses auf die Prostaglandin-Biosynthese wurde Avena sativa L. auch ohne Samen (2,5 g/100 ml) wie oben beschrieben, extrahiert (s. Abb. 1).

Anstelle von Wasser (destilliertem Wasser) kann auch ein Wasser-Alkohol-Gemisch verwendet werden.

Beispiel 2 (für Ultrafiltrate)

Avena sativa L., 0,25 g und 2,5 g werden mit 100 ml heißem Wasser übergossen und 10 Minuten unter mehrfachem Umrühren bedeckt stehen gelassen. Nach der Filtration wird die wäßrige Lösung einer Membranfiltration mit einer Ausschlußgrenze 1000 MG unterzogen (gesammeltes Diafiltrat 1500 ml, Filtrationsdruck 4,5 bar, $N_2$). Danach wurde das Diafiltrat auf ein Volumen von 100 ml eingeengt. Die weitere Behandlung erfolgte wie unter Beispiel 1, Absatz 2, angegeben. Die Wirkungen dieses Ultrafiltrats sind in Abb. 1,3,5 dargestellt.

Beispiel 3 (für TSK-Säulen-Fraktionen)

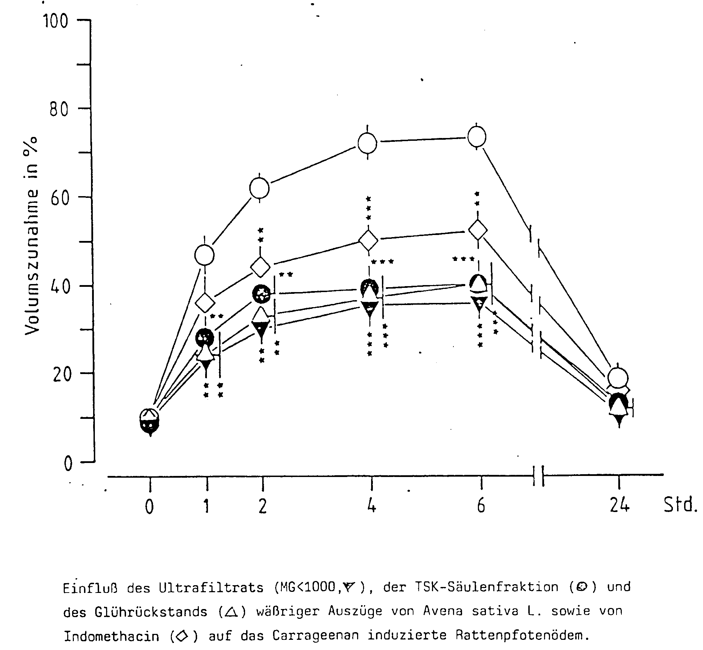

Das wie in Beispiel 2 erhaltene Ultrafiltrat (2,5 g Avena sativa L. mit 100 ml Wasser) wurde über TSK-Säulen (Dim. 2,5 x 50 cm, Elutionsbereich $V_e$ 100-140 ml) fraktioniert. Die Wirkung ist in Abb. 5 dargestellt.

Beispiel 4 (für Glührückstand)

2,5 g von Avena sativa L. wurden über 2 Stunden bei 800°C verascht. Die Asche wurde in 100 ml Wasser aufgenommen, mit Methylzellulose (Endkonzentration 1 %) versetzt und zu einer feinen Suspension verrührt. Die Wirkung ist in Abb. 5 dargestellt.

b) Experimentelle Durchführung

Prostaglandin Biosynthese

Für die Bestimmung der Prostaglandin-Biosynthese werden Stücke eines Rattenmagens (glandulärer Teil) 38 $\pm$ 4 mg verwendet (jeweils 5 Stücke pro Rattenmagen, n = 4 Tiere). Sprague-Dawley Ratten SPF, weiblich und männlich, 180 - 200 g (Versuchstierzucht und -haltung, Himberg) wurden durch eine Überdosis von Pentobarbital i.p. getötet. Die Adaptation erfolgte in einem vollklimatisierten Raum (22 $\pm$ 2°C, 55 $\pm$ 5% rel. Luftfeuchtigkeit $12/12^n$ Hell/Dunkel, Leitungswasser, Standardfutter "Taco" Tagger, Graz; 3 Tiere/Makrolonkäfig Typ III) über eine Woche.

Die Prüfung der Avena sativa L. Extrakte auf Hemmung der Prostaglandin Biosynthese erfolgte an Magenstücken der Ratte mit einer $^{14}$C-labeling Methode nach ISAKSON et al. modifiziert nach JUAN und SAMETZ, Naunyn-Schmiedeberg Arch. Pharmacol. 3/4, 183-190(1980) in der folgenden Versuchsanordnung:

Stücke des Rattenmagens werden in 500 $\mu$l Tyrodelösung mit $^{14}$C-Arachidonsäure (1 $\mu$Ci) über 30 Minuten bei 37° inkubiert. Die $^{14}$C-markierte Arachidonsäure wird in das Gewebe aufgenommen. Nach einem Waschvorgang werden die Stücke über 60 min bei 37° C mit und ohne Avenaextraktzusatz inkubiert. Von den jeweiligen Extrakten (s. Abb. 1) werden 100 $\mu$l + 400 $\mu$l Tyrode (insgesamt wieder 500 $\mu$l Inkubationsflüssigkeit) eingesetzt. Die Kontrollen enthalten statt dessen 100 $\mu$l isotone Kochsalzlösung. Zu Vergleichszwecken wurde der bekannte Cyclooxygenase Hemmer Indomethacin (Na-Salz), 1 $\mu$g/ml, einer weiteren Gruppe zugesetzt.

Aus der in die Zellmembranen aufgenommenen $^{14}$C-Arachidonsäure entstehen spontan $^{14}$-C markierte Eicosanoide. Nach der Inkubation werden die einzelnen Proben mit Äthylacetat dreimal extrahiert. In dieser Fraktion sind die $^{14}$-C- markierten Eicosanoide enthalten. Nach entsprechender Einengung werden $^{14}$C-markierte Produkte mittels DC aufgetrennt (gleichzeitige Zugabe authentischer unmarkierter Produkte). Die einzelnen Zonen (0,4 cm) der gesamten DC-Laufbahn werden in Flüssigkeits-Szintillationszähler auf Radioaktivität vermessen, so daß die $^{14}$C- markierten Prostaglandine detektierbar werden.

Die verwendete Tyrodelösung hat die übliche Zusammensetzung. Sie enthält pro 1000 ml Aqua dest. die folgenden Stoffe:

| | | | |
|---|---|---|---|
| NaCl | 8.0 g | $Na^+$ | 149.2 mmol |
| KCl | 0.2 g | $K^+$ | 2.7 mmol |
| $CaCl_2 . 2 H_2O$ | 0.26 g | $Ca^{++}$ | 1.8 mmol |
| $MgCl_2 . 5 H_2O$ | 0.21 g | $Mg^{++}$ | 2.1 mmol |
| $NaHCO_3$ | 1.0 g | $HCO_3^-$ | 11.9 mmol |
| $NaH_2PO_4 . 2H_2O$ | 0.06 g | $H_2PO_4^-$ | 0.4 mmol |
| Glucose | 1.1 g | $Cl^-$ | 145.3 mmol |
| | | Glucose | 5.5 mmol |

Magenstücke der Ratte werden deshalb verwendet, weil sie relativ große Mengen an Prostaglandin $E_2$ und $I_2$

bilden. In Abb. 1 wird die Hemmung der Biosynthese bzw. Freisetzung dieser $^{14}$C- markierten Prostaglandine demonstriert. Prostaglandin $I_2$ wurde in Form seines stabilen Abbauproduktes 6-keto-Prostaglandin $F_{1\alpha}$ gemessen.

Crotonöltest

Wie im Fall der Magenstückentnahme erhielten die Ratten ab 15 Stunden vor Versuchsbeginn nur mehr Wasser ad libitum. Auch die Haltungsbedingungen waren ident. Eine halbe Stunde vor Versuchsbeginn wurden die Tiere mit Pentobarbital-Na (Nembutal$^R$) narkotisiert. Die Narkose wurde während des gesamten Versuchs durch kleine Nachgaben aufrecht erhalten.

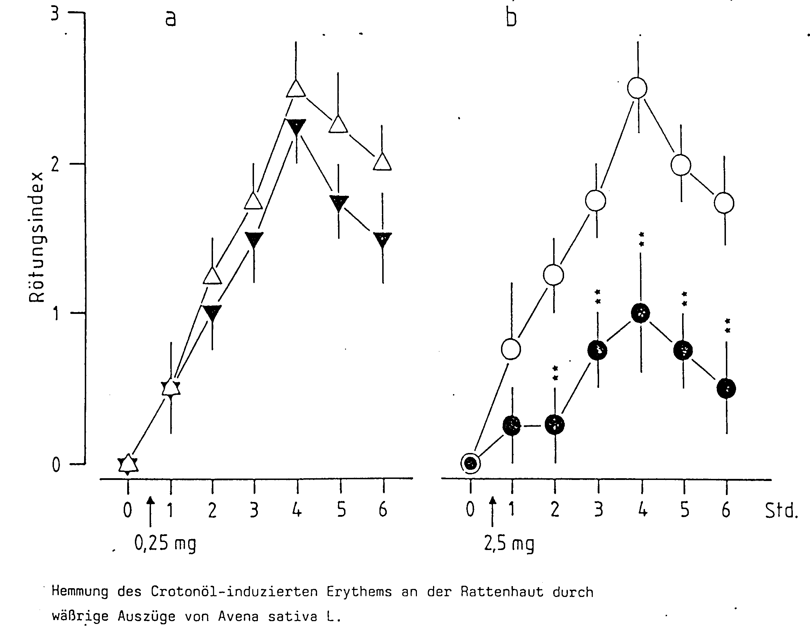

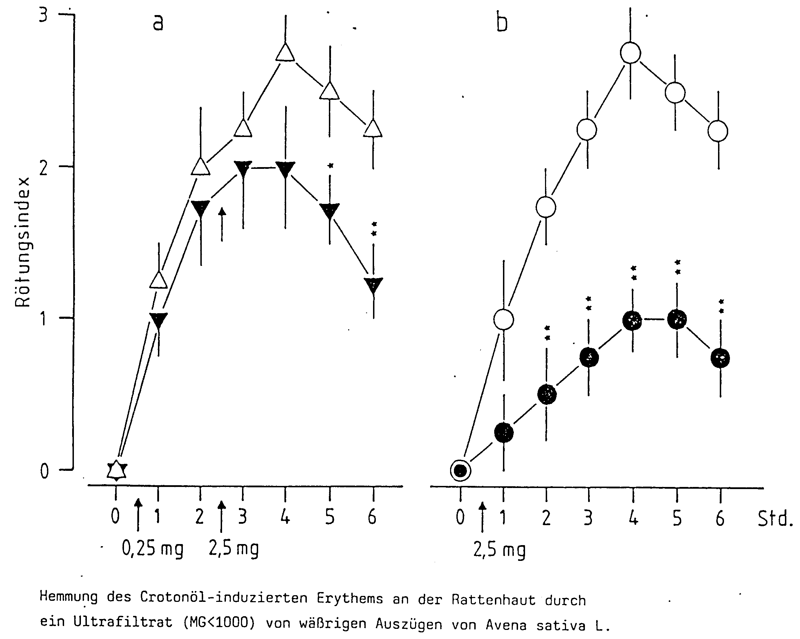

Für die Prüfung der lokalen, entzündungshemmenden Wirkung wurde der Crotonöltest verwendet. Crotonöl wurde mit Aceton verdünnt (Endkonzentration 5 mg/ml). Von dieser Konzentration wurden 20 µl (100 µg) mit einer Pipette auf einer Fläche von 0.6 cm$^2$ auf dem haarlosen Innenteil des Ohres aufgetragen. 30 min nach der Applikation von Crotonöl wurden die Avena-Extrakte (gemischt in 1 % Methylzellulose) in einer Menge von 100 µl auf die durch Crotonöl benetzte Fläche aufgebracht. Das kontralaterale Ohr diente als Positurkontrolle. Die Messung des Erythems erfolgte mit einer Rötungsskala (Semiquantitativ im Blindversuch) von 0= keine Rötung, 1 = schwach, 2 = mittel, 3 = stark. Für die Darstellung wurden die Werte (0-3) aller Tiere einer Gruppe (n=4) addiert und der Mittelwert berechnet. Die Ergebnisse sind in Abb.2 und 3 dargestellt.

Carrageenan Rattenpfoten-Ödem

Die Haltung und Adaptation der Ratten erfolgte wie oben beschrieben. Auch hier wurden die Tiere vor Versuchsbeginn mit Pentobarbital (Nembutal$^R$) narkotisiert, (50 mg/kg i.p.). Zwei Stunden und 30 Minuten vor Injektion von Carrageenan (0.1 ml, 2 %) in die Plantarregion der rechten Hinterpfote und Natriumchloridlösung (0.1 ml, 0,9%) in die linke Hinterpfote wurde eine Gruppe der jeweiligen Avena-Extrakte (10 ml/kg KG) oral (Schlundsonde) appliziert. Die Kontrollgruppen erhielten die entsprechende Menge an Wasser. Einige Gruppen erhielten zwei Stunden vor Carrageenangabe verschiedene Dosen von Indomethacin als Positivkontrollen und zum Wirkungsvergleich. Die wäßrigen Extrakte bzw. Fraktionen von Avena sativa L. wurden zweimal gegeben, da über die Kinetik des Wirkprinzips bzw. der Wirkprinzipien nichts bekannt ist. Auch die einmalige Gabe hemmt jedoch die Entwicklung des Ödems (keine Abbildung). Gemessen wird die Volumszunahme in %, bezogen auf das Volumen der Kontrollpfote (0 %). Der O-Std.-Wert ist die Messung direkt nach Carrageenan Gabe. Die Volumsveränderung wird mittels einer sehr genauen Quecksilberverdrängungsmethode über einen Druckmeßgeber bestimmt.

Statistik

Bei allen Ergebnissen der Untersuchungen wurden die Mittelwerte $\pm$ SEM berechnet. Zur Berechnung der Signifikanzen (p<0.05) wurden Student's t-Test oder die Varianzanalyse herangezogen.

Ergebnisse

1. Hemmender Einfluß auf die Prostaglandin-Biosynthese

Wie aus Abbildung 1 hervorgeht, hemmen verschiedene wäßrige Auszüge von Avena sativa L., 100 µl in 500 µl Tyrode, die Biosynthese von $PGE_2$ und $PGI_2$ in Rattenmagenstücken, ähnlich wie Indomethacin, 1 µg/ml.

Legende zu Abb. 1: Die $^{14}$C-markierten Prostaglandine $PGE_2$ und $PGI_2$ (gemessen als 6-keto-$PGF_{1\alpha}$) werden in counts per minute (kpm)/mg Gewebe gemessen. K = Kontrolle; E = wäßriger Extrakt (2,5 g/100 ml Wasser); U = Ultrafiltrat MG < 1000; W = wäßriger Auszug von Avena sativa L. ohne Samen; I = Indomethacin, 1 µg/ml.

Die Endkonzentration von 100 µl der Extrakte in 500 µl Tyrodelösung entspricht 5 mg Droge in 1 ml.
n = 4; $\bar{x}\pm$ SEM, $^{***}$p<0.001.

Abb. 1 stellt den Nachweis der Hemmung der Prostaglandinbiosynthese gemäß Beispiel 1, 2 und 3 dar.

2. Hemmender Einfluß auf das Crotonöl Erythem der Rattenhaut

Wie aus den Abbildungen 2 und 3 hervorgeht, hemmen verschiedene wäßrige Auszüge von Avena sativa L. bei lokaler Applikation dosisabhängig die durch Crotonöl auf der Rattenhaut induzierte Rötung. Eine bereits bestehende Rötung geht nach lokaler Gabe rasch wieder zurück. Dies bedeutet, daß Avena sativa L. sowohl prophylaktisch als auch therapeutisch (im engeren Sinn) wirksam ist.

Legende zu Abb. 2: Wirkung eines wäßrigen Avena-Extraktes (lokale Gabe, s. Pfeile) auf die durch Crotonöl (5 mg/ml in Aceton, davon 20 µl) an der Haut der Ratte (0.6 cm$^2$ der Ohr-Innenfläche) induzierten Rötung (Rötungsindex 0 - 3).

a) △ = Kontrolle; ▼ = wäßriger Extrakt, 100 μl von 0,25 g/100 ml $H_2O$.
b) ◯ = Kontrolle; ● = wäßriger Extrakt, 100 μl von 2,5 9/100 ml $H_2O$.

Die Konzentration in 100 μl Extrakt entspricht 2,5 mg (a.)9 bzw. 25 mg (b.)) Droge in 1 ml.
n = 4; x̄± SEM, [x]p<0.05, [xx]p<0.01
Legende zu Abb. 3: Wirkung eines Ultrafiltrats (MG<1000) des Avena-Extraktes (lokale Gabe, s. Pfeile) auf die durch Crotonöl an der Haut der Ratte induzierten Rötung.

a) △ = Kontrolle; ▼ = 100 μl des Ultrafiltrats aus einem wäßrigen Extrakt von 0.25 g/100 ml $H_2O$. 150 Minuten nach Crotonölauftragung wurden zusätzlich 100 μl des Ultrafiltrats, aus einem wäßrigen Extrakt von 2.5 g/100 ml $H_2O$, aufgetragen (s. Pfeil).

b) ◯ = Kontrolle; ● = 100 μl des Ultrafiltrats aus einem wäßrigen Extrakt von 2,5 g/100 ml $H_2O$.

n = 4; x̄±SEM, [x]p<0.05, [xx]p<0.01
Die Abbildungen 2 und 3 stellen den Nachweis der Hemmung des Crotonöl-Erythems bei lokaler Gabe (cutan) gemäß Beispiel 1 und 2 dar.

3. Hemmender Einfluß auf das Carrageenan induzierte Rattenpfotenödem

a) Wäßrige Auszüge

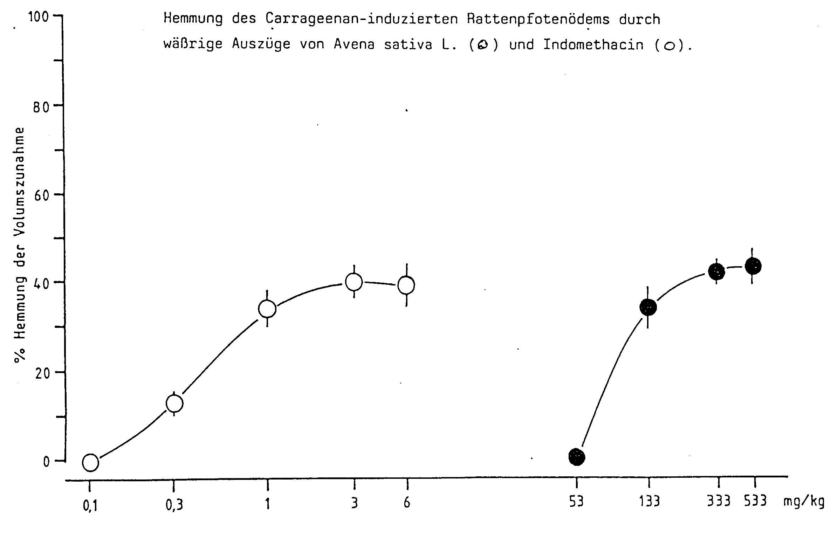

Wie aus Abbildung 4 hervorgeht, hemmen verschiedene wäßrige Auszüge von Avena sativa L. bei oraler Gabe dosisabhängig die Entwicklung des Pfotenödems. Die maximal wirksame Dosis erreicht ungefähr dieselbe maximale Wirksamkeit wie das bekannte Antirheumatikum Indomethacin, wie ein Vergleich der Dosis-Wirkungskurven zeigt.
Legende zu Abb. 4: Carrageenan Pfotenödem der Ratte. Einfluß mehrerer Dosen von wäßrigen Avena - Extrakten und von Indomethacin bei oraler Gabe auf die Entwicklung des Pfotenödems, ausgedrückt in Hemmung der Volumszunahme der Kontrollpfote in Prozent. Die angegebenen Dosen des Avena-Extraktes wurden zweimal (120 und 30 min vor Carrageenan), die von Indomethacin einmal (120 min vor Carrageenan) gegeben.

◯ = Indomethacin
● = Wäßrige Avena-Extrakte, jeweils 10 ml/kg eines Extraktes von 0.8 g/150 ml (entspricht 53 mg Droge/kg KG), 2 g/150 ml (133mg/kg), 5 g/150 ml (333 mg/kg) und 8 g/150 ml (533 mg/kg).
n = 5; x̄±SEM.

Abbildung 4 stellt den Nachweis der Hemmung einer akuten experimentellen Entzündung bei oraler Gabe gemäß Beispiel 1 dar.

b) Ultrafiltrat (MG<1000), TSK-Säulenfraktion und Glührückstand

Wie Abbildung 5 zeigt, hemmen das Ultrafiltrat (MG<5000), eine TSK-Säulenfraktion und ein Glührückstand (800°C) des wäßrigen Auszugs von Avena sativa L. (2,5 g Droge in 100 ml Wasser) bei oraler Gabe die Entwicklung des Pfotenödems. Die Wirkung dieser Extrakte bzw. Fraktionen war stärker als die des vergleichsweise gegebenen Indomethacins bei 1 mg/kg.
Legende zu Abb. 5: Carrageenan Pfotenödem der Ratte. Einfluß des Ultrafiltrats (MG<1000), der TSK-Säulenfraktion und des Glührückstands (2 Std. bei 800°C) eines wäßrigen Avena-Extraktes (2,5 g/100 ml $H_2O$, davon je 10ml/kg KG) bei oraler Gabe auf die Entwicklung des Pfotenödems. Die Dosis wurde zweimal (120 und 30 min vor Carrageenan Gabe) appliziert, die von Indomethacin einmal / 120 min vor Carrageenan). Gemessen wurde die Volumszunahme in Prozent (s. Methode).
◯ = Kontrolle; ▼= Ultrafiltrat; ● = TSK-Säulenfraktion; △ = Glührückstand; ◊ = Indomethacin, 1 mg / kg.
Die Dosis in 10 ml/kg der Extrakte entspricht 250 mg Droge pro. kg KG
n = 5, x̄±SEM, [xx]p<0.01, [xxx]p<0.001.
Abb. 5 stellt den Nachweis der Hemmung einer akuten experimentellen Entzündung bei oraler Gabe des Ultrafiltrats, der TSK-Säulenfraktion und des Glührückstands eines wäßrigen Avena-Extraktes gemäß Beispiel 2,3 und 4 dar. Indomethacin erwies sich in der gegebenen Dosis von 1 mg/kg als schwächer wirksam.

Zusammenfassung

Die Ergebnisse zeigen, daß verschiedene, erfindungsgemäße Extrakte von Avena sativa L. die Biosynthese von Prostaglandinen hemmen. In den verwendeten Dosen bzw. Konzentrationen sind die Effekte mit denen des bekannten Antirheumatikums Indomethacin vergleichbar. Wirksam sind wäßrige Extrakte der Gesamtpflanze ohne Samen. Ebenso wirksam ist auch das Ultrafiltrat mit einem MG unter 1000.
Die entzündungshemmende Wirkung bei lokaler Anwendung an der Haut geht klar aus dem Einfluß wäßriger Avena-Extrakte und des Ultrafiltrats auf das Crotonöl-induzierte Erythem hervor. Auch hier zeigt sich, daß das Wirkprinzip ein Molekulargewicht von unter 1000 aufweist.
An einem Modell einer akuten Entzündung (Carrageenan-induziertes Pfotenödem der Ratte) erweisen sich wäßrige Auszüge von Avena sativa L., das Ultrafiltrat (MG<1000) und eine TSK-Säulenfraktion bei oraler Gabe als stark antiphlogistisch. Die entzündungshemmende Wirkung ist im Hinblick auf das Maximum durchaus mit der von Indomethacin vergleichbar. Das Molekulargewicht des Wirkprinzips liegt wiederum unter 1000. Fraktionen mit höherem MG waren unwirksam (keine Darstellung).
Auch Glührückstände von Avena sativa L. (2 Std. bei 800°C) hemmen die akute experimentelle Entzündung. In diesem Fall könnte es sich durchaus um ein anderes Wirkprinzip, unterschiedlich von wirksamen Inhaltsstoffen der wäßrigen Extrakte, handeln.
Wenn vorstehend von Eicosanoiden gesprochen ist, so ist dieser Begriff im engeren Sinne von Prostaglandinen zu verstehen. Die entzündungshemmenden Effekte kommen sowohl bei innerer als auch bei äußerer Anwendung zum Tragen. Die Tierexperimente wurden im Rahmen des vom Österreichischen Bundesministerium für Wissenschaft und Forschung bewilligten Projektes GZ 205/323-14/89 durchgeführt.

## Patentansprüche

1.  Verwendung von Fraktionen aus Extrakten der Gesamtpflanze Avena sativa L. ohne Samen mit einem Molekulargewicht der Inhaltsstoffe kleiner als 1000 zur Herstellung von Arzneimitteln zur Hemmung entzündlicher Prozesse.

2.  Verwendung gemäß Anspruch 1, dadurch gekennzeichnet, daß als Extrakt ein wässriger Auszug verwendet wird.

3.  Verwendung gemäß Anspruch 1, dadurch gekennzeichnet, daß aus dem Extrakt ein Ultrafiltrat hergestellt wird.

4.  Verwendung gemäß Anspruch 3, dadurch gekennzeichnet, daß aus dem Ultrafiltrat eine TSK-Säulenfraktion hergestellt wird.

## Claims

1.  Use of fractions from extracts of the whole plant Avena sativa L. without seeds with a molecular weight of the constituent substances of less than 1000 for the production of drugs to inhibit inflammatory processes.

2.  Use according to Claim 1, characterised in that an aqueous extraction is used as the extract.

3.  Use according to Claim 1, characterised in that an ultrafiltrate is produced from the extract.

4.  Use according to Claim 3, characterised in that a TSK column fraction is produced from the ultrafiltrate.

## Revendications

1.  Utilisation de fractions tirées des extraits de la plante totale Avena sativa L. sans graine, ayant un poids moléculaire des ingrédients plus petit que 1000, en vue de la préparation de médicaments pour l'inhibition des processus inflammatoires.

2.  Utilisation selon la revendication 1,
    caractérisée en ce qu'
    on utilise en tant qu'extrait une fraction aqueuse.

3.  Utilisation selon la revendication 1,
    caractérisée en ce qu'
    on prépare à partir de l'extrait un produit d'ultrafiltration.

4. Utilisation selon la revendication 3,
   caractérisée en ce qu'
   on prépare à partir du produit d'ultrafiltration une fraction de colonne TSK.

Hemmung der Biosynthese von $PGI_2$ und $PGE_2$ durch wäßrige Auszüge und ein Ultrafiltrat von Avena sativa L.. Vergleich mit Indomethacin.

Hemmung des Crotonöl-induzierten Erythems an der Rattenhaut durch
wäßrige Auszüge von Avena sativa L.

Hemmung des Crotonöl-induzierten Erythems an der Rattenhaut durch ein Ultrafiltrat (MG<1000) von wäßrigen Auszügen von Avena sativa L.

EP 0 531 735 B1

Hemmung des Carrageenan-induzierten Rattenpfotenödems durch wäßrige Auszüge von Avena sativa L. (⌀) und Indomethacin (○).

EP 0 531 735 B1

Einfluß des Ultrafiltrats (MG<1000,▼), der TSK-Säulenfraktion (◉) und des Glührückstands (△) wäßriger Auszüge von Avena sativa L. sowie von Indomethacin (◇) auf das Carrageenan induzierte Rattenpfotenödem.